(19) Europäisches Patentamt
European Patent Office
Office européen des brevets

(11) **EP 4 635 484 A1**

(12) **EUROPEAN PATENT APPLICATION**
published in accordance with Art. 153(4) EPC

(43) Date of publication:
**22.10.2025 Bulletin 2025/43**

(21) Application number: **24744955.6**

(22) Date of filing: **17.01.2024**

(51) International Patent Classification (IPC):
***A61K 9/16*** *(2006.01)* ***A61K 31/00*** *(2006.01)*
***A61K 9/50*** *(2006.01)* ***A61P 29/00*** *(2006.01)*

(52) Cooperative Patent Classification (CPC):
**A61K 9/16; A61K 9/50; A61K 31/00; A61P 29/00**

(86) International application number:
**PCT/KR2024/095027**

(87) International publication number:
**WO 2024/155178 (25.07.2024 Gazette 2024/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **20.01.2023 KR 20230008905**
**17.10.2023 KR 20230138673**

(71) Applicant: **Inventage Lab Inc.**
**Gyeonggi-do 13403 (KR)**

(72) Inventors:
- **KIM, Ju Hee**
  **Seongnam-si, Gyeonggi-do 13494 (KR)**
- **SEO, Young Dai**
  **Seongnam-si, Gyeonggi-do 13494 (KR)**

(74) Representative: **BCKIP Part mbB**
**MK1**
**Landsbergerstraße 98, 3.Stock**
**80339 München (DE)**

(54) **MICROPARTICLES CONTAINING CANNABIDIOL AND MANUFACTURING METHOD THEREFOR**

(57) The present invention relates to microparticles containing cannabidiol. A problem may arise in that stability is reduced due to oxidation of cannabidiol contained in the microparticles. According to the present invention, it is possible to increase stability by preventing oxidation of cannabidiol in the microparticles. Moreover, the present invention relates to a method for producing microparticles that include cannabidiol and a biodegradable polymer, contain cannabidiol uniformly therein, and have a uniform particle size.

**FIG. 1**

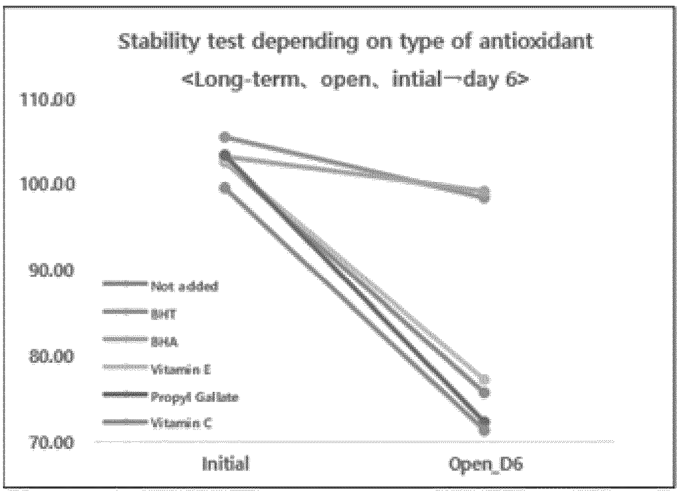




EP 4 635 484 A1

## Description

### Technical Field

The present invention relates to microparticles containing cannabidiol and a method for producing the same.

### Background Art

Cannabis is also called hemp, and its scientific name is *Cannabis sativa L.* The straight root thereof extends 30 to 40 cm underground, but the lateral roots do not develop vigorously, so they are easily pulled out. Cannabis is used for fabrics, mosquito nets, ropes, fishing nets, and papermaking materials. Hemp fruits are squeezed to obtain oil, which is used for food, lamp oil, soap, varnish, and paint, and the cake remaining after squeezing the oil is used as feed and fertilizer.
In particular, the resin extracted from the hemp hair is called hashish and has the effect of causing hallucinations. When this is dried, it becomes cannabis. Representative physiologically active components of cannabis include cannabidiol (CBD) and tetrahydrocannabinol (THC).
Cannabidiol (CBD) is one of the main components of cannabis and is a substance that is often compared to tetrahydrocannabinol (THC). In Korea, cannabidiol is designated as a narcotic and has not been studied a lot, but in foreign countries, cannabidiol is actively used for medical purposes to alleviate symptoms such as pain, memory impairment, and anxiety, and has been actively studied.
Cannabidiol has been known to have various effects through previous studies, and development into various cannabidiol formulations for increasing convenience of administration has been made.
In order to use cannabidiol for the above-described medicinal purposes, it is necessary to develop a formulation that may increase convenience by exhibiting a long-term medication effect through a single injection.

[Prior Art Documents]

[Patent Documents]

KR 10-2107715 B1

## DISCLOSURE

### Technical Problem

An object of the present invention is to provide microparticles containing cannabidiol and a method for producing the same.
Another object of the present invention is to provide microparticles containing cannabidiol, which has increased stability by preventing oxidation of cannabidiol in the microparticles, since a problem may arise in that stability is reduced due to oxidation of cannabidiol contained in the microparticles.
Still another object of the present invention is to provide a method for producing microparticles containing cannabidiol uniformly therein and having a uniform particle size.

### Technical Solution

To achieve the above objects, the present invention relates to microparticles containing cannabidiol, which may include cannabidiol (CBD), a sustained-release agent, and an antioxidant.
The sustained-release agent may be a biodegradable polymer.
The biodegradable polymer may be selected from the group consisting of polylactic acid, polylactide, polylactic-co-glycolic acid, polylactide-co-glycolide (PLGA), polyphosphazine, polyiminocarbonate, polyphosphoester, polyanhydride, polyorthoester, polycaprolactone, polyhydroxyvalerate, polyhydroxybutyrate, polyamino acids, and combinations thereof.
The antioxidant may be selected from the group consisting of butylated hydroxytoluene (BHT), butylhydroxyanisole (BHA), vitamin C, vitamin E, propyl gallate, and mixtures thereof.
The antioxidant may be included in an amount of 0.11 wt% to 9.9 wt% based on the total weight of cannabidiol and the sustained-release agent.
A method for producing microparticles containing cannabidiol according to another embodiment of the present invention may include steps of: preparing an oil-phase solution by dissolving cannabidiol (CBD), a sustained-release agent, and an antioxidant in an organic solvent; preparing an aqueous-phase solution by dissolving a surfactant in water;

and mixing the oil-phase solution and the aqueous-phase solution to form an emulsion.

The method may further include steps of: collecting the formed emulsion in the aqueous-phase solution to remove residual solvent; and washing and freeze-drying the emulsion from which the residual solvent has been removed.

The antioxidant may be selected from the group consisting of butylated hydroxytoluene (BHT), butylhydroxyanisole (BHA), vitamin C, vitamin E, propyl gallate, and mixtures thereof.

The antioxidant may be included in an amount of 0.06 wt% to 9.9 wt% based on the total weight of cannabidiol and the sustained-release agent.

The oil-phase solution and the aqueous-phase solution may be injected into the respective microchannels and allowed to flow therethrough, and the emulsion including cannabidiol, the antioxidant and the sustained-release agent may be formed at a point where the flow of the oil-phase solution and the flow of the aqueous-phase solution intersect each other.

Advantageous Effects

The present invention provides microparticles containing cannabidiol. A problem may arise in that stability is reduced due to oxidation of cannabidiol contained in the microparticles. According to the present invention, it is possible to increase stability by preventing oxidation of cannabidiol in the microparticles.

The present invention also provides a method for producing microparticles containing cannabidiol uniformly therein and having a uniform particle size.

## Brief Description of Drawings

FIG. 1 shows the results of measuring the encapsulation efficiency of cannabidiol depending on the type of antioxidant in microparticles according to one example of the present invention.

FIG. 2 shows the results of measuring the encapsulation efficiency of cannabidiol depending on the type of antioxidant in microparticles according to one example of the present invention.

FIG. 3 shows the results of measuring the encapsulation efficiency of cannabidiol depending on the content range of an antioxidant in microparticles according to one example of the present invention.

FIG. 4 shows the results of measuring the encapsulation efficiency of cannabidiol depending on the content range of an antioxidant in microparticles according to one example of the present invention.

FIG. 5 shows the results of measuring the encapsulation efficiency of cannabidiol depending on the content range of an antioxidant in microparticles according to one example of the present invention.

FIG. 6 shows the results of measuring the encapsulation efficiency of cannabidiol depending on the content range of an antioxidant in microparticles according to one example of the present invention.

Best Mode

Hereinafter, embodiments of the present invention will be described in detail so that those skilled in the art can easily implement the present invention. However, the present invention may be embodied in various different forms and is not limited to the embodiments described herein.

Cannabidiol that is used in the present invention is a physiologically active substance isolated from cannabis. It may be extracted directly from cannabis, or may be extracted cannabidiol, or may be produced by chemical synthesis, without being limited to the above examples.

Cannabis is also called hemp, and was used as cloth in the past. More specifically, hemp fibers are used for fabrics, mosquito nets, ropes, fishing nets, and papermaking materials. Hemp fruits are squeezed to obtain oil, which is used for food, lamp oil, soap, varnish, and paint, and the cake remaining after squeezing the oil is used as feed and fertilizer.

In countries where cannabis is classified as an illegal drug, storage and handling without special permission are considered as illegal acts. Cannabis, which is designated as an illegal drug, is also called marijuana or hemp and has been classified and managed as the most dangerous drug.

The World Health Organization (WHO) published a report stating that cannabidiol oil based on a cannabis extract is effective against epilepsy, Alzheimer's disease (dementia), and the like.

The World Anti-Doping Agency has published the "2018 International Standard for Prohibited Lists" and excluded cannabidiol from the list of prohibited drugs starting this year. Cannabidiol is a drug widely used by athletes for pain treatment. The use of other substances that may be obtained from cannabis, such as hashish and marijuana, have been banned, but cannabidiol oil for medical purposes has been permitted.

As mentioned above, the use of cannabidiol for medical purposes is permitted in various countries.

In addition, cannabidiol has been scientifically proven to be effective for various diseases such as epilepsy, Alzheimer's disease (dementia), allergic asthma, anxiety and sleep disorders, blood pressure improvement, diabetes, inflammation, irritable bowel disease, and multiple seizures.

Based on the therapeutic effects described above, formulations containing cannabidiol in various forms have been developed.

Specifically, the present invention relates to microparticles including cannabidiol (CBD), a sustained-release agent, and an antioxidant.

The microparticles including the sustained-release agent as described above, when injected *in vivo,* may exhibit the effect of releasing cannabidiol in a sustained manner for a long period of time by degradation of the sustained-release agent.

Cannabidiol has been approved for use for medical purposes as described above, but strict management is required in cases of administration, etc. Considering this point, the microparticles of the present invention are for use as an injectable formulation and are suitable for administering cannabidiol for medical purposes.

In addition, the microparticles may increase the convenience of administration by eliminating the inconvenience of repeated administration since they may exhibit a long-term sustained-release effect by a single administration.

When a conventional pharmaceutical formulation is used for diseases currently known to be effectively alleviated by cannabidiol, it is difficult for the pharmaceutical formulation to exhibit the effect of treating or alleviating the disease by a single administration.

That is, cannabidiol is considered applicable to a disease for which it may exhibit the effect of alleviating or treating symptoms by continuous repeated administration.

Accordingly, when the microparticles of the present invention are administered by injection, they may exhibit the effect of administering cannabidiol in a sustained manner by a single administration. Specifically, the microparticles may maintain the blood concentration of cannabidiol at the effective concentration or higher for one day by a single administration, may maintain the blood concentration of cannabidiol at the effective concentration or higher for several days by a single administration, may maintain the blood concentration of cannabidiol at the effective concentration or higher for one week by a single administration, may maintain the blood concentration of cannabidiol at the effective concentration or higher for several weeks by a single administration, may maintain the blood concentration of cannabidiol at the effective concentration or higher for one month by a single administration, may maintain the blood concentration of cannabidiol at the effective concentration or higher for several months by a single administration, may maintain the blood concentration of cannabidiol at the effective concentration or higher for three month by a single administration, may maintain the blood concentration of cannabidiol at the effective concentration or higher for 6 months by a single administration, or may maintain the blood concentration of cannabidiol at the effective concentration or higher for 12 months by a single administration.

That is, the microparticles may exhibit the effect of releasing cannabidiol in a sustained manner for 1 day or more, 1 week or more, 1 month or more, 3 months or more, 6 months or more, or 12 months or more.

The effect of releasing cannabidiol in a sustained manner as described above may be affected by the degradation rate of the sustained-release agent. The sustained-release agent will be described in detail below.

However, the microparticles containing cannabidiol and the sustained-release agent have a problem in that oxidation of cannabidiol occurs. As described below, after cannabidiol is formulated into microparticles, it can be confirmed that cannabidiol in the microparticles is changed to yellow by oxidation.

Cannabidiol may be a compound represented by the following formula:

[Formula]

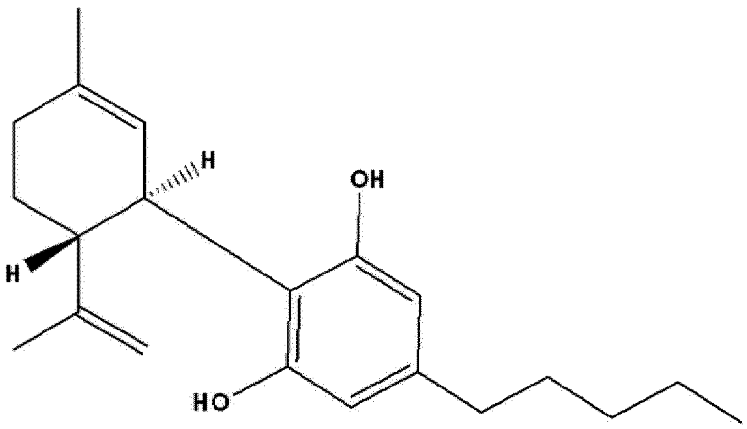

Cannabidiol, a compound represented by the formula above, is a stable compound that is not oxidized by contact with oxygen in the air.

However, when cannabidiol is formulated into microparticles including the sustained-release agent as disclosed in the present invention, the cannabidiol may be oxidized.

If cannabidiol is oxidized as described above, its color may be changed to yellow as described above, and as the structure of the compound changes due to oxidation, a decrease in the efficacy thereof may occur.

Accordingly, the present invention is characterized by including an antioxidant to prevent oxidation of cannabidiol in the microparticles.

The antioxidant is selected from the group consisting of butylhydroxytoluene (BHT), butylhydroxyanisole (BHA), vitamin C, vitamin E, propyl gallate, and mixtures thereof, and is selected from the group consisting of butylhydroxytoluene (BHT), butylhydroxyanisole (BHA), and mixtures thereof, and may be butylhydroxytoluene (BHT) or butylhydroxyanisole (BHA).

The antioxidant can prevent oxidation of cannabidiol by being included in the microparticles. That is, the antioxidant generally refers to a substance that prevents oxidation. Since the antioxidant is oxidized instead of cannabidiol in the microparticles, it may prevent oxidation of cannabidiol.

However, even if any type of antioxidant is included in the microparticles, it does not prevent the oxidation of cannabidiol, and the effect of preventing the oxidation of cannabidiol may appear only when the antioxidant described above is included.

That is, the antioxidant is generally a substance that prevents oxidation, and as described above, it may prevent cannabidiol from being oxidized by being oxidized before cannabidiol.

The microparticles of the present invention are spherical microparticles as described below, and when the solvent is completely removed therefrom through the production process, cannabidiol, the sustained-release agent, and the antioxidant may be uniformly distributed therein.

In this case, in an environment where the microparticles come into contact with oxygen, an oxidation reaction of cannabidiol occurs, but an oxidation reaction of the antioxidant occurs before the oxidation reaction of cannabidiol, so that the oxidation reaction of cannabidiol may be prevented.

Although the oxidation reaction of cannabidiol may be prevented by the above-described action, not all antioxidants exhibit this antioxidative effect, and only the antioxidant of the present invention may exhibit the effect of preventing the oxidation of cannabidiol by being oxidized before cannabidiol. That is, in the case of microparticles including an antioxidant other than the antioxidant of the present invention, cannabidiol may be oxidized before the antioxidant.

More specifically, among the antioxidants of the present invention, only an antioxidant selected from the group consisting of butylhydroxytoluene (BHT), butylhydroxyanisole (BHA), and a mixture thereof may exhibit the effect of preventing the oxidation of cannabidiol by being oxidized before cannabidiol. Other antioxidants do not exhibit the antioxidative effect because cannabidiol is oxidized before the antioxidants.

The antioxidant may be included in an amount of 0.11 wt% to 9.9 wt%, 0.2 wt% to 9 wt%, 0.3 wt% to 8 wt%, or 0.5 wt% to 5 wt%, based on the total weight of cannabidiol and the sustained-release agent. Within the above range, it is possible to produce spherical microparticles, the microparticles may be used as a sustained-release formulation by maintaining an appropriate content range of cannabidiol, and it is possible to increase stability through the effect of the antioxidant that prevents the oxidation of cannabidiol.

The biodegradable polymer is selected from the group consisting of polylactic acid, polylactide, polylactic-co-glycolic acid, polylactide-co-glycolide (PLGA), polyphosphazine, polyiminocarbonate, polyphosphoester, polyanhydride, polyorthoester, polycaprolactone, polyhydroxyvalerate, polyhydroxybutyrate, polyamino acids, and combinations thereof, and is preferably polylactide-co-glycolide (PLGA) or polylactide (PLA), but is not limited to the above examples.

As an example, the molar ratio of glycolide to lactide in the polylactide-co-glycolide may be about 60:40 to about 90:10, about 60:40 to about 85:15, about 60:40 to about 80:20, about 60:40 to about 75:25, about 65:35 to about 90:10, about 70:30 to about 90:10, about 75:25 to about 90:10, about 65:35 to about 85:15, or about 70:30 to about 80:20, without being limited to the above examples. Preferably, the molar ratio of glycolide to lactide in the polylactide-co-glycolide may be about 75:25.

The biodegradable polymer may include one or more types of polylactide and one or more types of polylactide-co-glycolide. In the present invention, the biodegradable polymer may include, for example, two types of polylactide, a combination of one type of polylactide and one type of polylactide-co-glycolide, two types of polylactide-co-glycolide, three types of polylactides, a combination of two types of polylactide and one type of polylactide-co-glycolide, a combination of one type of polylactide and two types of polylactide-co-glycolide, or the like, and in particular, may include a combination of one type of polylactide and one type of polylactide-co-glycolide, or two types of polylactide-co-glycolide, without being not limited thereto.

The biodegradable polymer may include two or more types of polylactide-co-glycolide.

The microparticles may include cannabidiol and the biodegradable polymer at a weight ratio of 1:1 to 1:10, 1:2 to 1:8, 1:2 to 1:6, or 1:4. When the microparticles including cannabidiol and the biodegradable polymer at a weight ratio within the above range are used as an injectable composition, they may be used as a sustained-release injectable composition that releases cannabidiol in a sustained manner within an injectable dose range. That is, if the weight ratio of cannabidiol to the biodegradable polymer is lower than the lower limit of the above range, the amount of the injectable composition will be increased to meet the total dose for administering cannabidiol by injection, which causes a problem of exceeding the amount of the injectable composition that may be administered once to a person. In addition, if the weight ratio of cannabidiol to the biodegradable polymer is higher than the upper limit of the above range, it will not be easy to produce microparticles in which cannabidiol is uniformly distributed.

A sustained-release injectable composition including cannabidiol according to another embodiment of the present invention may include: the microparticles containing cannabidiol; and a suspending solvent.

The injectable composition may include a suspending solvent, wherein the suspending solvent includes an isotonic agent, a suspending agent, and a solvent.

More specifically, the isotonic agent may be selected from the group consisting of D-mannitol, maltitol, sorbitol, lactitol, xylitol, sodium chloride, and mixtures thereof, and is preferably D-mannitol, without being limited to the above examples.

The suspending agent is selected from the group consisting of sodium carboxymethylcellulose, polysorbate 80, starch, starch derivatives, polyhydric alcohols, chitosan, chitosan derivatives, cellulose, cellulose derivatives, collagen, gelatin, hyaluronic acid (HA), alginic acid, algin, pectin, carrageenan, chondroitin, chondroitin sulfate, dextran, dextran sulfate, polylysine, titin, fibrin, agarose, fluran, xanthan gum, and mixtures thereof, and is preferably sodium carboxymethylcellulose and polysorbate 80, without being limited to the above examples.

As the solvent, water for injection may be used, and any solvent that may be used as water for injection may be used without limitation.

A method for producing microparticles containing cannabidiol according to still another embodiment of the present invention may include steps of: preparing an oil-phase solution by dissolving cannabidiol (CBD), a sustained-release agent and an antioxidant in an organic solvent; preparing an aqueous-phase solution by dissolving a surfactant in water; and mixing the oil-phase solution and the aqueous-phase solution to form an emulsion.

According to the production method, an emulsion may be formed using an oil-phase solution prepared by dissolving cannabidiol, a sustained-release agent, and an antioxidant in an organic solvent and an aqueous-phase solution containing a surfactant.

As described below, according to the present invention, the emulsion is produced by a microfluidic method using a microchannel, without being limited to this production method, and any microparticle production method such as a solvent evaporation method or a membrane method may be applied.

The organic solvent may be one capable of completely dissolving cannabidiol, the sustained-release agent and the antioxidant. Specifically, the organic solvent may be any one or more selected from the group consisting of chloroform, chloroethane, dichloroethane, dichloromethane, trichloroethane, methylene chloride, methanol, and mixtures thereof, and preferably may be selected from the group consisting of methylene chloride, methanol, and mixtures thereof. In addition to the above-listed organic solvents, any organic solvent may be used without limitation, as long as it is capable of completely dissolving cannabidiol, the sustained-release agent and the antioxidant and may be easily selected by those skilled in the art.

The cannabidiol and sustained-release agent in the organic solvent may be contained at a weight ratio of 1:1 to 1:10, 1:2 to 1:8, 1:2 to 1:6, or 1:4.

The sustained-release agent in the oil-phase solution is contained in an amount of 10 to 20 wt%, preferably 12 to 18 wt%, more preferably 15 wt%, without being to the above examples. If the sustained-release agent is contained in an amount smaller than the lower limit of the above range, it is impossible to prepare an emulsion, and if the sustained-release

agent is contained in an amount larger than the upper limit of the above range, there is a problem in that the viscosity of the oil-phase solution is excessively high, making it not easy to prepare an emulsion.

The biodegradable polymer is selected from the group consisting of polylactic acid, polylactide, polylactic-co-glycolic acid, polylactide-co-glycolide (PLGA), polyphosphazine, polyiminocarbonate, polyphosphoester, polyanhydride, polyorthoester, polycaprolactone, polyhydroxyvalerate, polyhydroxybutyrate, polyamino acids, and combinations thereof, and is preferably polylactide-co-glycolide (PLGA) or polylactide (PLA), without being limited to the above examples.

The aqueous-phase solution may contain the surfactant in an amount of 0. 1 to 1.0 wt%, 0.2 to 0.5 wt%, or 0.25 wt%, with the remainder being water.

The surfactant may be any one or more selected from the group consisting of methylcellulose, polyvinylpyrrolidone, lecithin, gelatin, polyvinyl alcohol, sorbitan monooleate (e.g., Span™ 80, etc.), polyoxyethylene sorbitan fatty acid ester (e.g., Tween™ 80, etc.), polyoxyethylene castor oil derivatives, sodium lauryl sulfate, sodium stearate, ester amines, linear diamines, patty amines, and mixtures thereof, and preferably may be polyvinyl alcohol, without being limited to the above examples, and any surfactant that may be prepared into a perfectly spherical emulsion may be used.

After the oil-phase solution and the aqueous-phase solution are separately prepared as described above, the method of preparing an emulsion using the same is not limited.

However, in the present invention, a method of producing microparticles using a microfluidic method will be described.

A microchip for using the microfluidic method may be formed on a wafer or a glass substrate. Microchannels are formed in the microchip. More specifically, the microchannels include a channel through which the oil-phase solution flows, a channel through which the aqueous-phase solution flows, and a transport channel. The channel through which the oil-phase solution flows and the channel through which the aqueous-phase solution flows are formed to meet each other at one point, and one end of the transport channel may be connected to a portion where the two channels are joined.

Injection portions for injecting the oil-phase solution and the aqueous-phase solution are connected to the channel through which the oil-phase solution flows and the channel through which the aqueous-phase solution flows, respectively, and a recovery portion for recovering a solution containing an emulsion may be connected to one end of the transport channel.

The microchannels may be formed on a material selected from the group consisting of a glass substrate, a silicon wafer and a polymer film, but the material is not limited to the above examples, and it is possible to use any material on which the microchannels may be formed.

The polymer film may be selected from the group consisting of polyimide, polyethylene, fluorinated ethylene propylene, polypropylene, polyethylene terephthalate, polyethylene naphthalate, polysulfone, and mixtures thereof, without being limited to the above examples.

As an example, aluminum is deposited on a silicon wafer using an e-beam evaporator, and photoresist is patterned on the aluminum using a photolithography technique. Thereafter, the aluminum is etched using the photoresist as a mask, and the photoresist is removed. Then, the silicon wafer is etched by deep ion reactive etching (DRIE) using the aluminum as a mask, the aluminum is removed, and then glass is anodically bonded onto the wafer and hermetically sealed, thereby fabricating the microchannels.

The microchannels have an average diameter of 60 to 150 μm, preferably 80 to 100 μm, without being limited to the above example. If microchannels having an average diameter smaller than the lower limit of the above range are used, an emulsion having an excessively small diameter may be produced, which may affect the release and *in vivo* absorption of the effective drug.

In addition, if the microchannels have an average diameter larger than the upper limit of the above range, the average size of the produced microparticles will exceed 120 μm, and foreign body sensation and pain may increase when the microparticles are administered by injection. As the diameter of the microchannels increases, the particle size distribution of the produced microparticles increases, making it difficult to produce microparticles with a uniform particle size.

In addition, the average diameter of the microchannels is closely related not only to the average diameter of the particles, but also to the ratio of the flow rates (μl/min) of the oil-phase solution and the aqueous-phase solution.

In addition, the cross-sectional width (w) and the cross-sectional height (d) of the microchannel are closely related to the average diameter (d') of the microparticles being produced. The cross-sectional width (w) of the microchannel is in a ratio range of 0.7 to 1.3 with respect to the average diameter (d') of the microparticles, and the cross-sectional height (d) of the microchannel is in a ratio range of 0.7 to 1.3 with respect to the average diameter (d') of the microparticles.

That is, when the average diameter (d') of the microparticles to be produced is determined, the lengths of the cross-sectional width (w) and cross-sectional height (d) of the microchannel should be set in a ratio range of 0.7 to 1.3 with respect to d' so that microparticles having a desired size may be produced.

In order to produce microparticles using the above-described microchip, the oil-phase solution may be injected into the channel through which the oil-phase solution flows, and the aqueous-phase solution may be injected into the

channel through which the aqueous-phase solution flows, thereby forming an emulsion at the portion where the two channels are joined.

When the oil-phase solution and the aqueous-phase solution are injected into the respective microchannels, the ratio of the flow rates of the oil-phase solution and the aqueous-phase solution may be 1:10 to 1:50, 1:15 to 1:40, 1:15 to 1:30, or 1:15 to 1:25. By controlling the ratio of the flow rates of the oil-phase solution and the aqueous-phase solution as described above, it is possible to produce microparticles having a uniform diameter.

The emulsion formed in the portion where the two channels are joined may be collected using the previously prepared aqueous phase solution, i.e., a mixed solution of the surfactant and water. The prepared aqueous-phase solution may be used to form an emulsion by being injected into the microchannel, and may also be used to prevent the aggregation of emulsion particles by being placed into a bath.

Residual organic solvent may be removed from the emulsion collected in the bath. The step of removing the residual organic solvent may be performed by stirring the emulsion at a predetermined temperature and a predetermined stirring speed to evaporate and remove the residual organic solvent present in the emulsion. Here, the stirring is performed under the following conditions: a first stirring step at 15 to 20°C for 50 to 70 minutes; a second stirring step at 20 to 40°C for 50 to 70 minutes; and a third stirring step at 40 to 60°C for 1 to 3 hours.

The stirring speed is the same for all of the first to third stirring steps, and the stirring speed may be 300 to 500 rpm, or 400 rpm.

As described above, the stirring temperature is gradually increased as the stirring process progresses. By increasing the temperature stepwise, it is possible to control the evaporation rate of the organic solvent present in the emulsion.

The temperature at which the oil-phase solution and the aqueous-phase solution flow through the microchannels is also 5 to 20°C, preferably 10°C. That is, after the solutions flow along the microchannels and intersect each other to produce an emulsion, the collected emulsion is maintained at a constant low temperature of 5 to 20°C until it is stirred in the first stirring step. Only when the emulsion preparation process is maintained at a low temperature, it is possible to produce and maintain spherical particles. That is, if a low-temperature condition is not used, a problem arises in that it is difficult to produce particles having a uniform spherical shape.

Thereafter, in the second stirring step, the temperature is increased gradually and the stirring time is increased so that the organic solvent present in the emulsion may be moved slowly to the surface and evaporated from the surface, thereby minimizing the effect of the evaporation of the organic solvent on the appearance of the emulsion. That is, if the organic solvent is rapidly evaporated, a problem may arise in that the surfaces of the finally produced microparticles are not smooth and pores are formed, due to the evaporation of the organic solvent. In order to prevent this problem, the evaporation rate of the organic solvent may be controlled by increasing the temperature gradually as described above and also increasing the stirring process time, and due to this control of the evaporation rate of the organic solvent, it is possible to control the surface appearance of the produced microparticles.

In the third stirring process, after the organic solvent in the emulsion is extracted with the external aqueous phase, the external aqueous phase is stirred at an increased temperature near the boiling point of the organic solvent, thereby removing the saturated organic solvent from the aqueous phase, thus facilitating the removal of residual organic solvent from the emulsion.

Lastly, a step of washing and drying the microparticles from which residual organic solvent has been removed is performed. In this step, the microparticles from which the organic solvent on the surface has been completely removed by stirring are washed several times with sterile filtered purified water to remove the surfactant remaining in the microparticles, and then freeze-dried.

The final produced microparticles are in a form in which cannabidiol is uniformly distributed in the microparticles made of the spherical biodegradable polymer, and may include cannabidiol and the biodegradable polymer at a weight ratio of 1:1 to 1:10.

In addition, as described above, in the final produced microparticles, the antioxidant is uniformly distributed in addition to cannabidiol, and thus when the microparticles come into contact with oxygen in the air, the antioxidant may be preferentially oxidized, thereby preventing the oxidation of cannabidiol.

The weight ratio between cannabidiol and the biodegradable polymer contained in the microparticles is the same as the weight ratio in the oil-phase solution used to prepare the emulsion. Specifically, as the solutions pass through the microchannels to form the emulsion and the organic solvent is completely removed from the emulsion, it is possible to produce microparticles containing cannabidiol and the biodegradable polymers at the same weight ratio as the weight ratio in the oil-phase solution.

The microparticles may have an average diameter of 30 to 70 μm, 30 to 65 μm, or 30 to 60 μm. In addition, the standard deviation for the average diameter may be 1 to 30 μm, 1 to 20 μm, 1 to 10 μm, or 1 to 7 μm. It can be confirmed that it is possible to produce uniform particles within the above diameter range. When the uniform particles are used as a sustained-release injectable composition, they may reduce foreign body sensation, increase the convenience of administration, prevent initial over-release upon *in vivo* injection, and exhibit the effect of releasing cannabidiol in a sustained

manner.

Production Example 1

Production of Microparticles

Using the components and contents shown in Table 1 below, microparticles were produced in the following manner.

1) Preparation of Oil-Phase Solution

An oil-phase solution was prepared by dissolving a lactide-glycolide copolymer (75:25) and cannabidiol, or a lactide-glycolide copolymer (75:25), cannabidiol, and an antioxidant in methylene chloride or a mixture of methylene chloride and methanol. Here, the content of the polymer in the oil-phase solution was 15 wt%, and the weight ratio between the polymer and cannabidiol was 4:1.

2) Preparation of Aqueous-Phase Solution

An aqueous-phase solution was prepared by dissolving polyvinyl alcohol in water. The content of polyvinyl alcohol in the aqueous-phase solution was 0.25 wt%.

3) Production of Microparticles

The oil-phase solution and the aqueous-phase solution were injected into microchannels formed on a silicon wafer, thereby producing microparticles. Here, the ratio of the flow rate of the oil-phase solution to the flow rate of the aqueous-phase solution was 1:20, and the temperature was maintained at 10.0°C. The produced microparticles were collected in a bath containing the aqueous-phase solution, and stirred at a speed of 400 rpm at 10.0°C for 1 hour, at 30.0°C for 1 hour, and at 45.0°C for 2 hours.

4) Washing and Collection

After completion of the stirring, the microparticles were washed with sterile filtered purified water, freeze-dried, and then collected in powder form.

[Table 1]

| Purpose of mixing | Component name | Comp. Example 1 | Example 1 | Example 2 | Example 3 | Example 4 | Example 5 |
|---|---|---|---|---|---|---|---|
| | | Quantity (mg) | | | | | |
| Active ingredient | Cannabidiol | 100.00 | 100.00 | 100.00 | 100.00 | 100.00 | 100.00 |
| Sustained-release agent | Lactide-glycolide copolymer (75:25) | 400.00 | 400.00 | 400.00 | 400.00 | 400.00 | 400.00 |
| Antioxidant | Butylhydroxytoluene | - | 1.00 | - | - | - | - |
| | Butylhydroxyanisole | - | - | 1.00 | - | - | - |
| | Tocopherol acetate | - | - | - | 1.00 | - | - |
| | Propyl gallate | - | - | - | - | 1.00 | - |
| | Ascorbic acid | - | - | - | - | - | 1.00 |
| Organic solvent | Methylene chloride | 2,660.00 | 2,660.00 | 2,660.00 | 2,660.00 | 2,560.00 | 2,160.00 |
| Organic solvent | Methanol | - | - | - | - | 100.00 | 500.00 |

(continued)

| Purpose of mixing | Component name | Comp. Example 1 | Example 1 | Example 2 | Example 3 | Example 4 | Example 5 |
|---|---|---|---|---|---|---|---|
| | | Quantity (mg) | | | | | |
| Total mass (mg) | | 500.00 | 500.00 | 500.00 | 500.00 | 500.00 | 500.00 |

Production Example 2

Production of Microparticles

Using the components and contents shown in Table 2 below, microparticles were produced in the following manner.

1) Preparation of Oil-Phase Solution

An oil-phase solution was prepared by dissolving a lactide-glycolide copolymer (75:25), cannabidiol, and butylhydroxytoluene in methylene chloride. Here, the content of the polymer in the oil-phase solution was 15 wt%, and the weight ratio between the polymer and cannabidiol was 4: 1.

2) Preparation of Aqueous-Phase Solution

An aqueous-phase solution was prepared by dissolving polyvinyl alcohol in water. The content of polyvinyl alcohol in the aqueous-phase solution was 0.25 wt%.

3) Production of Microparticles

The oil-phase solution and the aqueous-phase solution were injected into microchannels formed on a silicon wafer, thereby producing microparticles. Here, the ratio of the flow rate of the oil-phase solution to the flow rate of the aqueous-phase solution was 1:20, and the temperature was maintained at 10.0°C. The produced microparticles were collected in a bath containing the aqueous-phase solution, and stirred at a speed of 400 rpm at 10.0°C for 1 hour, at 30.0°C for 1 hour, and at 45.0°C for 2 hours.

4) Washing and Collection

After completion of the stirring, the microparticles were washed with sterile filtered purified water, freeze-dried, and then collected in powder form.

[Table 2]

| Purpose of mixing | Component name | Comp. Example 2 | Example 6 | Example 7 | Example 8 | Example 9 | Example 10 | Comp. Example 3 |
|---|---|---|---|---|---|---|---|---|
| | | Quantity (mg) | | | | | | |
| BHT content (w/w%) | | 10% | 5% | 3% | 1% | 0.50% | 0.10% | 0.05% |
| Active ingredient | Cannabidiol | 100.00 | 100.00 | 100.00 | 100.00 | 100.00 | 100.00 | 100.00 |
| Sustained-release agent | Lactide-glycolide copolymer (75:25) | 400.00 | 400.00 | 400.00 | 400.00 | 400.00 | 400.00 | 400.00 |
| Antioxidant | Butylhydroxytoluene | 10.00 | 5.00 | 3.00 | 1.00 | 0.50 | 0.10 | 0.05 |
| Organic solvent | Methylene chloride | 2,660.00 | 2,660.00 | 2,660.00 | 2,660.00 | 2,660.00 | 2,660.00 | 2,660.00 |

(continued)

| Purpose of mixing | Component name | Comp. Example 2 | Example 6 | Example 7 | Example 8 | Example 9 | Example 10 | Comp. Example 3 |
|---|---|---|---|---|---|---|---|---|
| | | Quantity (mg) | | | | | | |
| Total mass (mg) | | 510.00 | 505.00 | 503.00 | 501.00 | 500.00 | 500.10 | 500.05 |

Production Example 3

Production of Microparticles

Using the components and contents shown in Table 3 below, microparticles were produced in the following manner.

1) Preparation of Oil-Phase Solution

An oil-phase solution was prepared by dissolving a lactide-glycolide copolymer (75:25), cannabidiol, and butylhydroxyanisole in methylene chloride. Here, the content of the polymer in the oil-phase solution was 15 wt%, and the weight ratio between the polymer and cannabidiol was 4: 1.

2) Preparation of Aqueous-Phase Solution

An aqueous-phase solution was prepared by dissolving polyvinyl alcohol in water. The content of polyvinyl alcohol in the aqueous-phase solution was 0.25 wt%.

3) Production of Microparticles

The oil-phase solution and the aqueous-phase solution were injected into microchannels formed on a silicon wafer, thereby producing microparticles. Here, the ratio of the flow rate of the oil-phase solution to the flow rate of the aqueous-phase solution was 1:20, and the temperature was maintained at 10.0°C.

The produced microparticles were collected in a bath containing the aqueous-phase solution, and stirred at a speed of 400 rpm at 10.0°C for 1 hour, at 30.0°C for 1 hour, and at 45.0°C for 2 hours.

4) Washing and Collection

After completion of the stirring, the microparticles were washed with sterile filtered purified water, freeze-dried, and then collected in powder form.

[Table 3]

| Purpose of mixing | Component name | Comp. Example 4 | Example 11 | Example 12 | Example 13 | Example 14 | Comp. Example 5 | Comp. Example 6 |
|---|---|---|---|---|---|---|---|---|
| | | Quantity (mg) | | | | | | |
| BHT content (w/w%) | | 10% | 5% | 3% | 1% | 0.50% | 0.10% | 0.05% |
| Active ingredient | Cannabidiol | 100.00 | 100.00 | 100.00 | 100.00 | 100.00 | 100.00 | 100.00 |
| Sustained-release agent | Lactide-glycolide copolymer (75:25) | 400.00 | 400.00 | 400.00 | 400.00 | 400.00 | 400.00 | 400.00 |
| Antioxidant | Butylhydroxyanisole | 10.00 | 5.00 | 3.00 | 1.00 | 0.50 | 0.10 | 0.05 |
| Organic solvent | Methylene chloride | 2,660.00 | 2,660.00 | 2,660.00 | 2,660.00 | 2,660.00 | 2,660.00 | 2,660.00 |

(continued)

| Purpose of mixing | Component name | Comp. Example 4 | Example 11 | Example 12 | Example 13 | Example 14 | Comp. Example 5 | Comp. Example 6 |
|---|---|---|---|---|---|---|---|---|
| | | Quantity (mg) | | | | | | |
| Total mass (mg) | | 510.00 | 505.00 | 503.00 | 501.00 | 500.50 | 500.10 | 500.05 |

Experimental Methods

Experimental Example 1

Stability Test

1) Sample Preparation

About 100 mg of microparticles were weighed and placed in a 5-mL glass vial which was then placed in a capped or uncapped (close/open) stability chamber.

2) Stability Test

The stability test was conducted under long-term conditions (temperature: 25±2°C, and relative humidity: 60 ±5%), and whether yellowing occurred was checked by observing a change in the appearance daily. Meanwhile, the initial sample and the sample on day 6 of placement in the stability chamber were tested for encapsulation efficiency to check whether the content changed.

Experimental Example 2

Encapsulation Efficiency Experiment

HPLC (high-performance liquid chromatography) was performed to measure the encapsulation efficiency (EE%), and the standard solution and test solution for analysis were prepared as follows.

1) Preparation of Standard Solution

About 10 mg of cannabidiol as an active ingredient was taken and placed in a 100-mL flask. About 50 mL of a diluent (acetonitrile) was added thereto and the mixture was sonicated for 10 minutes. After cooling sufficiently, the volume was adjusted to the mark with the diluent, and the resulting solution was filtered through a 0.45-μm filter (water-soluble PTFE) and then used as a standard solution (concentration: 0.1 mg/mL).

2) Preparation of Test Solution

About 50 mg of microparticles (10.0 mg as main ingredient) were taken and placed in a 100-mL flask. About 50 mL of a diluent (acetonitrile) was added thereto and the mixture was sonicated for 10 minutes. After cooling sufficiently, the volume was adjusted to the mark with the diluent, and the resulting solution was filtered through a 0.45-μm filter (water-soluble PTFE) and then used as a test solution (concentration: 0.1 mg/mL).

3) HPLC conditions

- Detector: UV (220 nm)
- Column: ODS column (4.6*150 mm, 5 μm)
- Flow rate: 1.0 mL/min
- Mobile phase: methanol: purified water = 85:15 (isocratic method)

4) Formula for calculation of encapsulation efficiency (EE%)

$$\text{Encapsulation efficiency (EE\%)} = At/As * Cs/Ct \times P$$

At: Area response of cannabidiol in test solution
As: Area response of cannabidiol in standard solution
Cs: Concentration of cannabidiol in standard solution
Ct: Concentration of cannabidiol in test solution
P: Purity of cannabidiol

Experimental Example 1

Production Example 1 is an experiment to evaluate the antioxidative effect of each antioxidant.

The experimental results are shown in FIGS. 7, 1 and 2.

Referring to the experimental results, it can be confirmed that the group to which no antioxidant was added turned yellow in a state in which the glass vial was completely closed or opened. In other words, it can be confirmed that cannabidiol in the microparticles was oxidized because an antioxidant was not included.

The above experimental results can be clearly confirmed through the encapsulation efficiency experiment and through the difference in values between the initial time point and day 6.

In addition, as a result of evaluating the antioxidative effect depending on the type of antioxidant, it can be observed with the naked eye that the effect of preventing the oxidation of cannabidiol appeared only when BHT or BHA was included. This can also be confirmed numerically in the encapsulation efficiency experiment.

Production Example 2 is an experiment to evaluate the antioxidative effect depending on the content of BHT as the antioxidant, and the experimental results are shown in FIGS. 8, 3 and 4.

Referring to the experimental results, it can be confirmed that the antioxidant effect was insignificant when BHT was contained in an amount of 0.05 wt% based on the total weight of cannabidiol and the biodegradable polymer.

In addition, it can be confirmed that, when BHT was contained in an amount of 0.1 wt% based on the total weight of cannabidiol and the biodegradable polymer, the color changed to yellow when observed with the naked eye, but the results of the encapsulation efficiency measurement showed that BHT exhibited the effect of suppressing the oxidation of cannabidiol.

In addition, it was confirmed that, when BHT was contained in an amount of 10 wt% based on the total weight of cannabidiol and the biodegradable polymer, a problem arose in that the microparticles agglomerated, and thus had unsuitable quality.

Production Example 3 is an experiment to evaluate the antioxidant effect depending on the content of BHA as the antioxidant, and the experimental results are shown in FIGS. 9, 5 and 6.

Referring to the experimental results, it can be confirmed that the antioxidant effect was insignificant when BHA was contained in an amount of 0.05 wt% based on the total weight of cannabidiol and the biodegradable polymer.

In addition, it can be confirmed that, when BHA was contained in an amount of 0.1 wt% based on the total weight of cannabidiol and the biodegradable polymer, the color changed to yellow when observed with the naked eye, but the results of the encapsulation efficiency measurement showed that BHA exhibited the effect of suppressing the oxidation of cannabidiol.

In addition, it was confirmed that, when BHA was contained in an amount of 10 wt% based on the total weight of cannabidiol and the biodegradable polymer, a problem arose in that the microparticles agglomerated, and thus had unsuitable quality.

Experimental Results 2

Results of Average Diameter Measurement

For the microparticles shown in FIGS. 7 to 9, the average diameter and the standard deviation of the average diameter were determined by PSA analysis, and the experimental results are shown in Tables 4 to 6 below.

[Table 4]

| | Not added | BHT 1% | BHA 1% | Vitamin E 1% | Propyl gallate 1% | Vitamin C 1% |
|---|---|---|---|---|---|---|
| Average diameter (mean ± SD) | 38.07±4.08 | 35.83±3.70 | 37.94±4.11 | 35.73±4.05 | 34.99±3.05 | 36.85±14.24 |

[Table 5]

| BHT | 10% | 5% | 3% | 1% | 0.50% | 0.10% | 0.05% |
|---|---|---|---|---|---|---|---|
| Average diameter (mean ± SD) | 39.67±14.79 | 34.98±3.87 | 35.52±3.90 | 36.05±4.66 | 35.75±4.38 | 34.67±4.86 | 34.73±3.39 |

[Table 6]

| BHA | 10% | 5% | 3% | 1% | 0.50% | 0.10% | 0.05% |
|---|---|---|---|---|---|---|---|
| Average diameter (mean ± SD) | 52.79±20.33 | 39.12±3.89 | 35.62±3.58 | 39.10±6.35 | 34.33±2.73 | 34.66±2.70 | 37.43±4.69 |

Referring to the above experimental results, the microparticles of the present invention may have an average diameter of 30 to 60 μm and a standard deviation of average diameter of 1 to 30 μm. In addition, it can be confirmed that the microparticles containing BHT or BHA as an antioxidant within the preferred content range of the present invention may have an average diameter of 30 to 40 μm and a standard deviation of average diameter of 1 to 7 μm, suggesting that the microparticles have a very uniform size distribution.

Although the preferred embodiments of the present invention have been described in detail above, the scope of the present invention is not limited thereto, and various modifications and improvements made by those skilled in the art using the basic concept of the present invention defined in the following claims also fall within the scope of the present invention.

## Mode for Invention

The present invention relates to microparticles including cannabidiol (CBD), a sustained-release agent, and an antioxidant.

Industrial Applicability

The present invention relates to microparticles containing cannabidiol and a method for producing the same.

## Claims

1. A microparticle containing cannabidiol, comprising cannabidiol (CBD), a sustained-release agent, and an antioxidant.

2. The microparticle containing cannabidiol according to claim 1, wherein the sustained-release agent is a biodegradable polymer.

3. The microparticle containing cannabidiol according to claim 2, wherein the biodegradable polymer is selected from the group consisting of polylactic acid, polylactide, polylactic-co-glycolic acid, polylactide-co-glycolide (PLGA), polyphosphazine, polyiminocarbonate, polyphosphoester, polyanhydride, polyorthoester, polycaprolactone, polyhydroxyvalerate, polyhydroxybutyrate, polyamino acids, and combinations thereof.

4. The microparticle containing cannabidiol according to claim 1, wherein the antioxidant is selected from the group

consisting of butylhydroxytoluene (BHT), butylhydroxyanisole (BHA), vitamin C, vitamin E, propyl gallate, and mixtures thereof.

**5.** The microparticle containing cannabidiol according to claim 1, wherein the antioxidant is comprised in an amount of 0.11 wt% to 9.9 wt% based on the total weight of cannabidiol and the sustained-release agent.

**6.** A method for producing microparticles containing cannabidiol, comprising steps of:

preparing an oil-phase solution by dissolving cannabidiol (CBD), a sustained-release agent, and an antioxidant in an organic solvent;
preparing an aqueous-phase solution by dissolving a surfactant in water; and
mixing the oil-phase solution and the aqueous-phase solution to form an emulsion.

**7.** The method for producing microparticles containing cannabidiol according to claim 6, further comprising steps of:

collecting the formed emulsion in the aqueous-phase solution to remove residual solvent; and
washing and freeze-drying the emulsion from which the residual solvent has been removed.

**8.** The method for producing microparticles containing cannabidiol according to claim 6, wherein the antioxidant is selected from the group consisting of butylhydroxytoluene (BHT), butylhydroxyanisole (BHA), vitamin C, vitamin E, propyl gallate, and mixtures thereof.

**9.** The method for producing microparticles containing cannabidiol according to claim 6, wherein the antioxidant is comprised in an amount of 0.06 wt% to 9.9 wt% based on the total weight of cannabidiol and the sustained-release agent.

**10.** The method for producing microparticles containing cannabidiol according to claim 6, wherein the oil-phase solution and the aqueous-phase solution are injected into the respective microchannels and allowed to flow therethrough, and the emulsion containing cannabidiol, the antioxidant and the sustained-release agent is formed at a point where the flow of the oil-phase solution and the flow of the aqueous-phase solution intersect each other.

FIG. 1

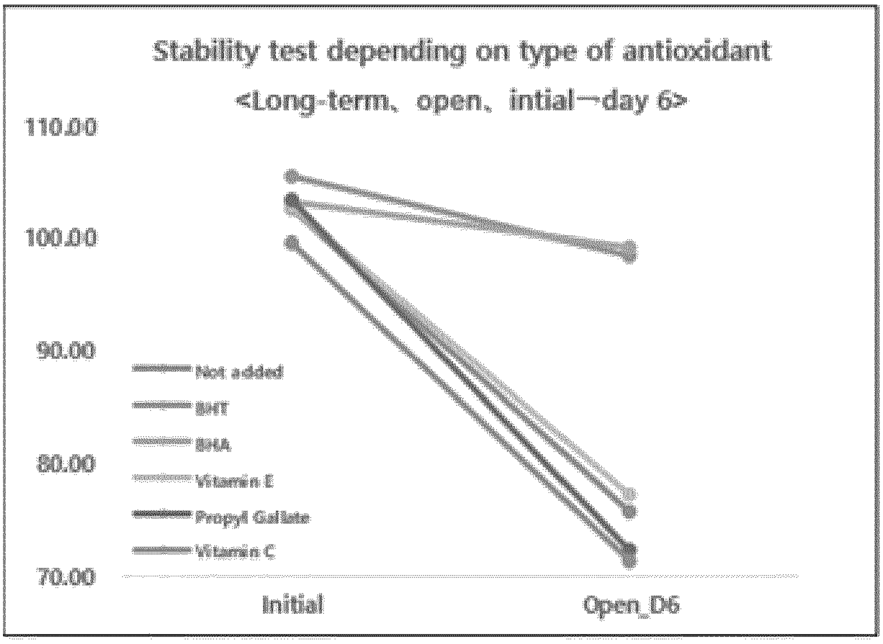
Stability test depending on type of antioxidant
<Long-term、open、intial→day 6>
110.00
100.00
90.00
80.00
70.00
Not added
BHT
BHA
Vitamin E
Propyl Gallate
Vitamin C
Initial
Open_D6

FIG. 2

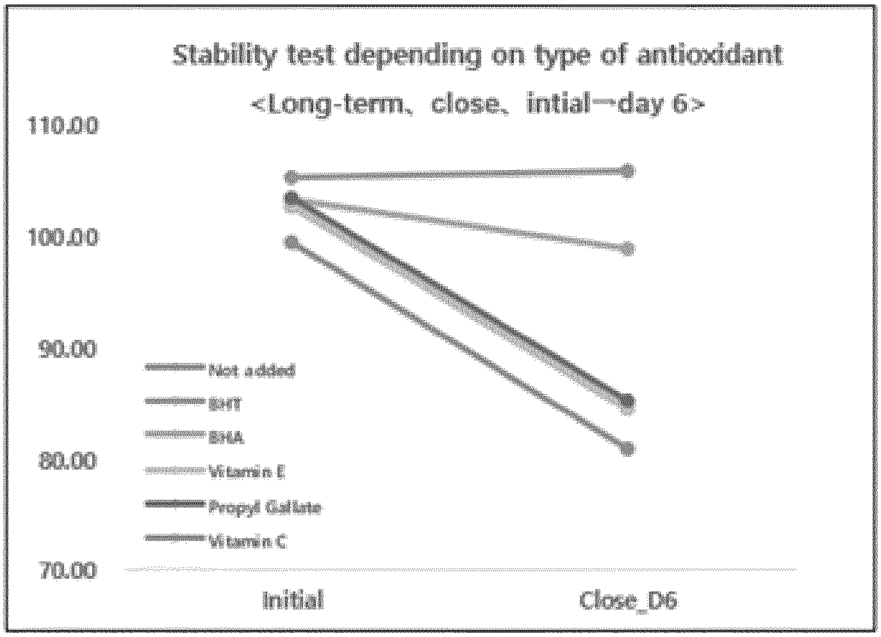
Stability test depending on type of antioxidant
<Long-term、close、intial→day 6>
110.00
100.00
90.00
80.00
70.00
Not added
BHT
BHA
Vitamin E
Propyl Gallate
Vitamin C
Initial
Close_D6

FIG. 3

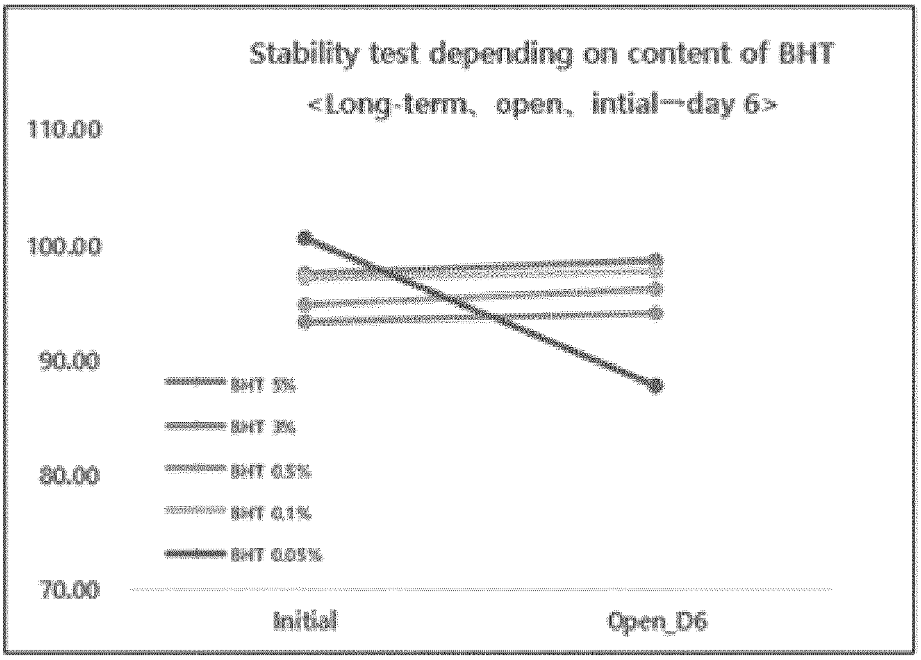
Stability test depending on content of BHT
<Long-term, open, intial→day 6>
110.00
100.00
90.00
80.00
70.00
BHT 5%
BHT 3%
BHT 0.5%
BHT 0.1%
BHT 0.05%
Initial
Open_D6

FIG. 4

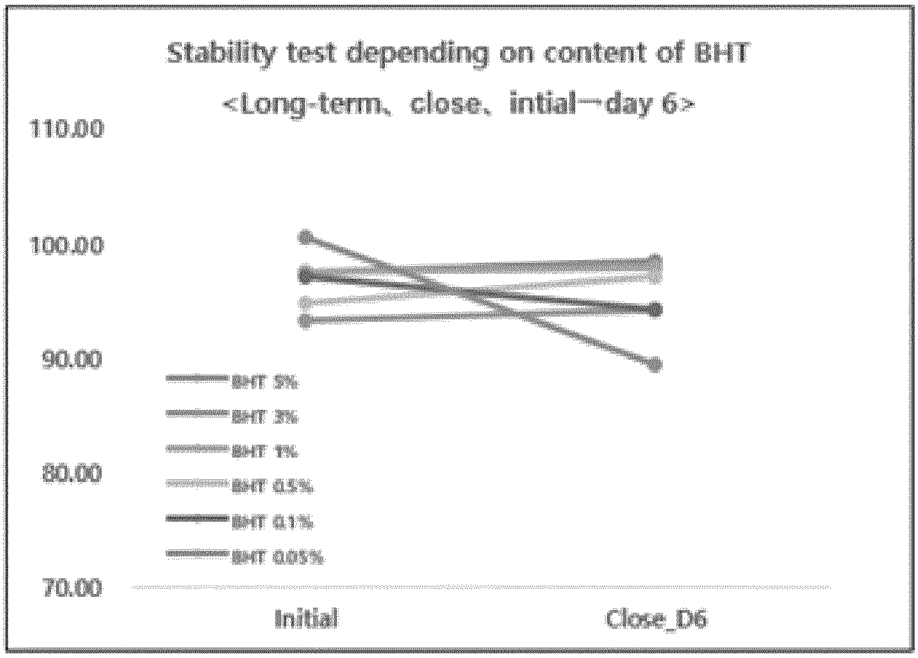
Stability test depending on content of BHT
<Long-term, close, intial→day 6>
110.00
100.00
90.00
80.00
70.00
BHT 5%
BHT 3%
BHT 1%
BHT 0.5%
BHT 0.1%
BHT 0.05%
Initial
Close_D6

FIG. 5

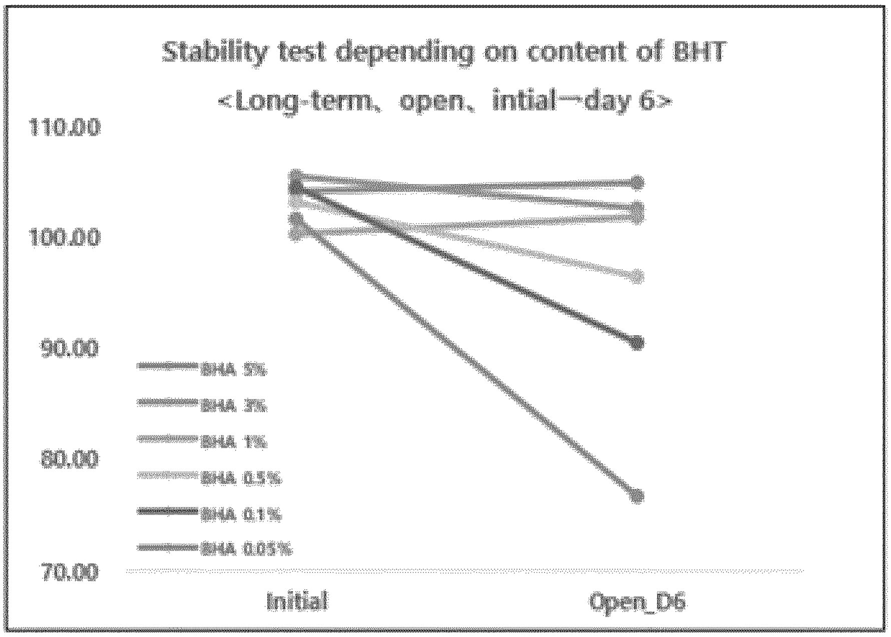
Stability test depending on content of BHT
<Long-term、open、intial→day 6>
110.00
100.00
90.00
80.00
70.00
BHA 5%
BHA 3%
BHA 1%
BHA 0.5%
BHA 0.1%
BHA 0.05%
Initial
Open_D6

FIG. 6

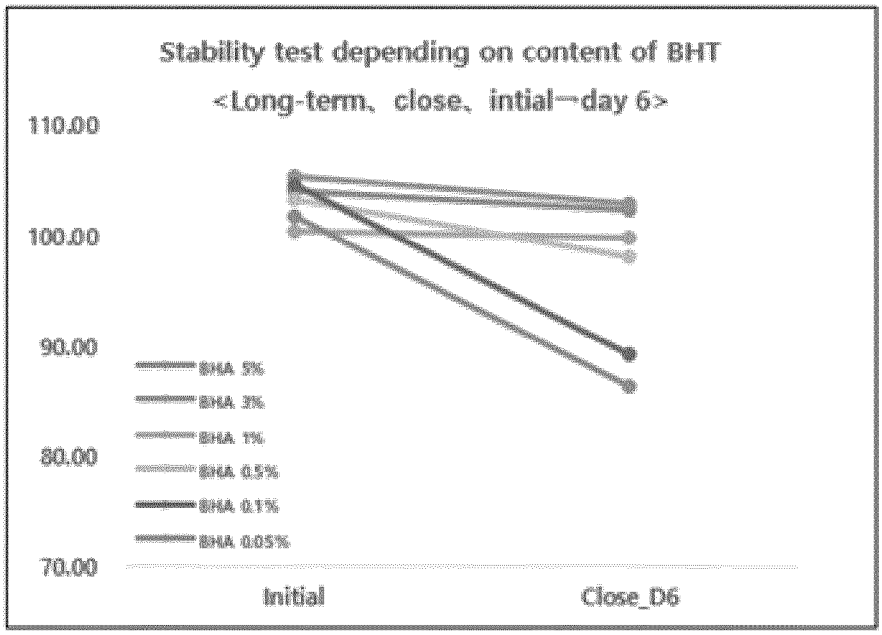
Stability test depending on content of BHT
<Long-term、close、intial→day 6>
110.00
100.00
90.00
80.00
70.00
BHA 5%
BHA 3%
BHA 1%
BHA 0.5%
BHA 0.1%
BHA 0.05%
Initial
Close_D6

FIG. 7

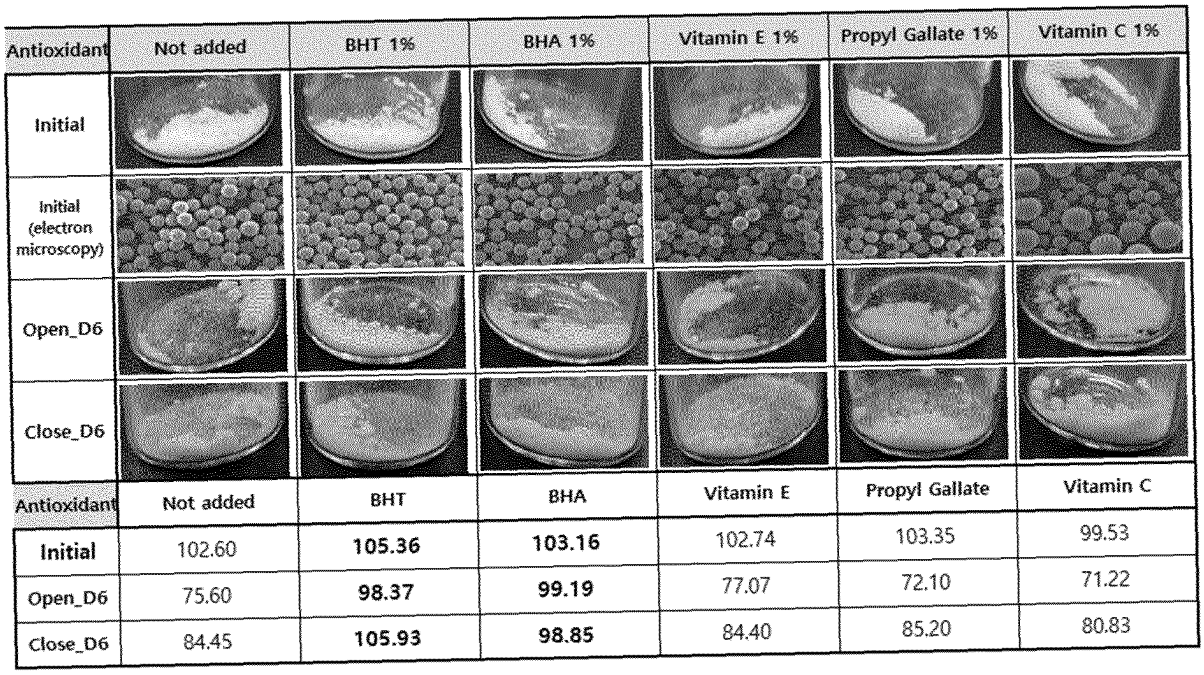


| Antioxidant | Not added | BHT | BHA | Vitamin E | Propyl Gallate | Vitamin C |
|---|---|---|---|---|---|---|
| **Initial** | 102.60 | **105.36** | **103.16** | 102.74 | 103.35 | 99.53 |
| Open_D6 | 75.60 | **98.37** | **99.19** | 77.07 | 72.10 | 71.22 |
| Close_D6 | 84.45 | **105.93** | **98.85** | 84.40 | 85.20 | 80.83 |

FIG. 8

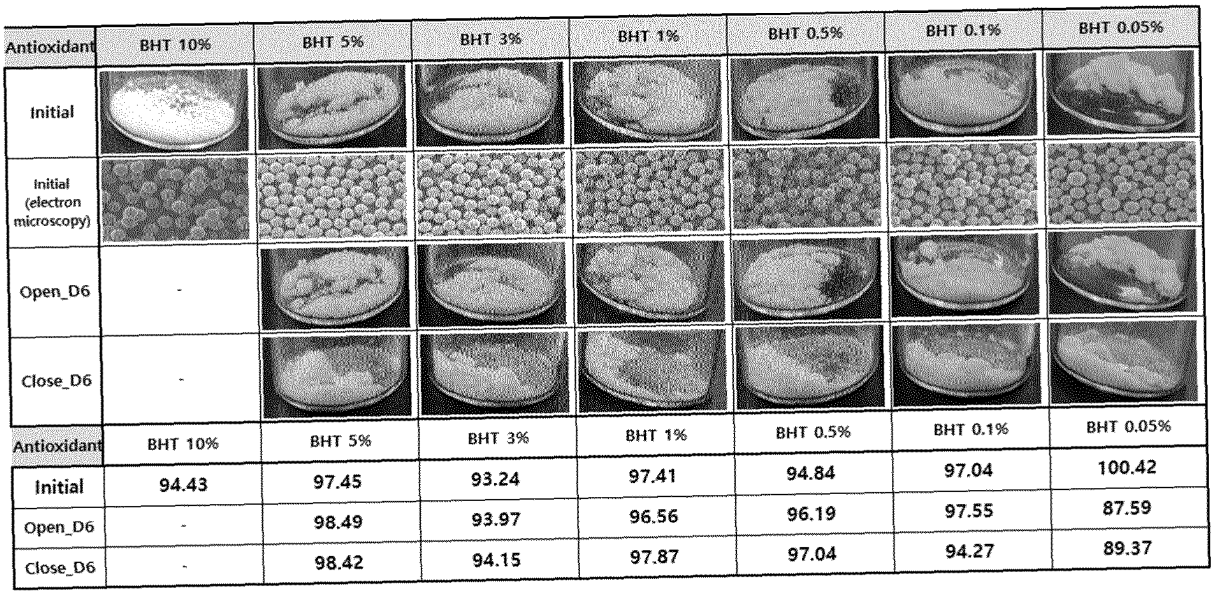


| Antioxidant | BHT 10% | BHT 5% | BHT 3% | BHT 1% | BHT 0.5% | BHT 0.1% | BHT 0.05% |
|---|---|---|---|---|---|---|---|
| Initial | 94.43 | 97.45 | 93.24 | 97.41 | 94.84 | 97.04 | 100.42 |
| Open_D6 | - | 98.49 | 93.97 | 96.56 | 96.19 | 97.55 | 87.59 |
| Close_D6 | - | 98.42 | 94.15 | 97.87 | 97.04 | 94.27 | 89.37 |

**FIG. 9**

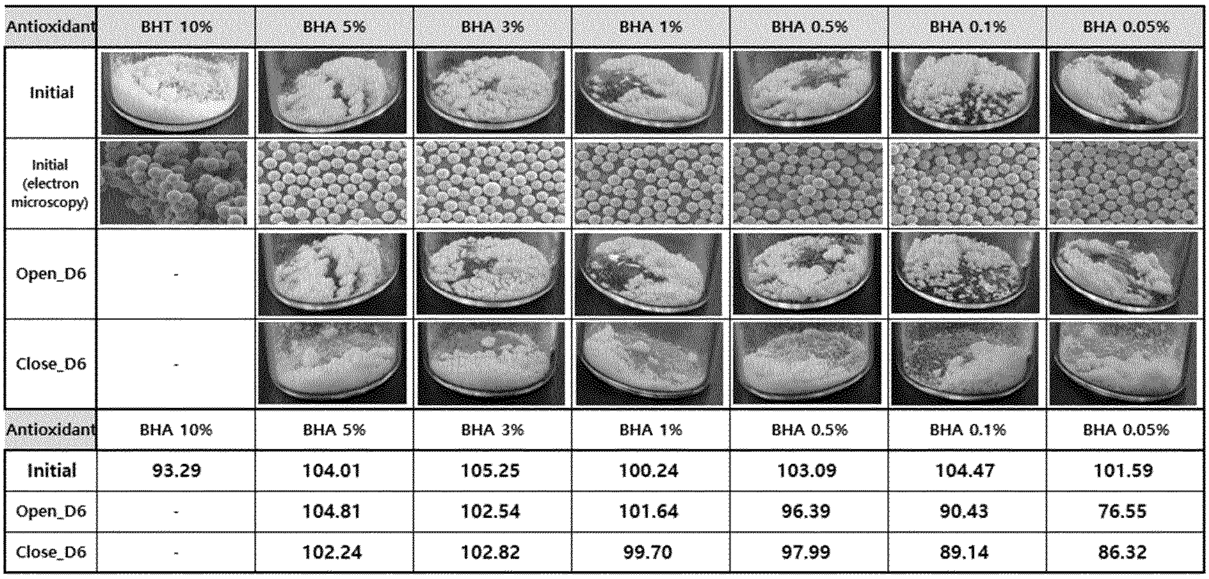


| Antioxidant | BHA 10% | BHA 5% | BHA 3% | BHA 1% | BHA 0.5% | BHA 0.1% | BHA 0.05% |
|---|---|---|---|---|---|---|---|
| Initial | 93.29 | 104.01 | 105.25 | 100.24 | 103.09 | 104.47 | 101.59 |
| Open_D6 | - | 104.81 | 102.54 | 101.64 | 96.39 | 90.43 | 76.55 |
| Close_D6 | - | 102.24 | 102.82 | 99.70 | 97.99 | 89.14 | 86.32 |

**INTERNATIONAL SEARCH REPORT**

International application No. **PCT/KR2024/095027**

**A. CLASSIFICATION OF SUBJECT MATTER**

**A61K 9/16**(2006.01)i; **A61K 31/00**(2006.01)i; **A61K 9/50**(2006.01)i; **A61P 29/00**(2006.01)i

According to International Patent Classification (IPC) or to both national classification and IPC

**B. FIELDS SEARCHED**

Minimum documentation searched (classification system followed by classification symbols)

A61K 9/16(2006.01); A61K 31/05(2006.01); A61K 47/06(2006.01); A61K 47/10(2006.01); A61P 25/08(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 칸나비디올(cannabidiol), 마이크로입자(microparticles), 생분해성 고분자(biodegradable polymer), 에멀젼(emulsion)

**C. DOCUMENTS CONSIDERED TO BE RELEVANT**

| Category* | Citation of document, with indication, where appropriate, of the relevant passages | Relevant to claim No. |
|---|---|---|
| Y | FRAGUAS-SÁNCHEZ, A. I. et al. Effect of Gamma Sterilization on CBD-Loaded PLGA Microparticles. Proceedings. 2021, vol. 78, document 31, pp. 1-6.<br>See abstract; and pages 1-2. | 1-10 |
| Y | KR 10-2022-0016147 A (GW RESEARCH LIMITED) 08 February 2022 (2022-02-08)<br>See abstract; and claims 1-2, 14-15 and 23-25. | 1-10 |
| A | JP 2022-552056 A (CARDIOL THERAPEUTICS INC.) 15 December 2022 (2022-12-15)<br>See entire document. | 1-10 |
| A | JP 2021-509667 A (GW RESEARCH LIMITIED) 01 April 2021 (2021-04-01)<br>See entire document. | 1-10 |
| A | KR 10-2020-0106049 A (GW RESEARCH LIMITED) 10 September 2020 (2020-09-10)<br>See entire document. | 1-10 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 April 2024** | **24 April 2024** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**
**Information on patent family members**

International application No.
**PCT/KR2024/095027**

| Patent document cited in search report | Publication date (day/month/year) | Patent family member(s) | Publication date (day/month/year) |
|---|---|---|---|
| KR 10-2022-0016147 A | 08 February 2022 | CA 3141987 A1 | 03 December 2020 |
| | | CN 113939283 A | 14 January 2022 |
| | | EP 3976004 A1 | 06 April 2022 |
| | | GB 2584341 A | 02 December 2020 |
| | | GB 2584341 B | 01 March 2023 |
| | | IL 288423 A | 01 January 2022 |
| | | JP 2022-534763 A | 03 August 2022 |
| | | US 2022-0233495 A1 | 28 July 2022 |
| | | WO 2020-240184 A1 | 03 December 2020 |
| JP 2022-552056 A | 15 December 2022 | AU 2019-465548 A1 | 03 March 2022 |
| | | BR 112022002409 A2 | 26 April 2022 |
| | | CA 3152020 A1 | 18 March 2021 |
| | | EP 4028059 A1 | 20 July 2022 |
| | | MX 2022002548 A | 22 March 2022 |
| | | US 2022-0347118 A1 | 03 November 2022 |
| | | WO 2021-046628 A1 | 18 March 2021 |
| JP 2021-509667 A | 01 April 2021 | CA 3087125 A1 | 11 July 2019 |
| | | CN 111757729 A | 09 October 2020 |
| | | CN 111757729 B | 08 August 2023 |
| | | EP 3735230 A1 | 11 November 2020 |
| | | GB 2572125 A | 25 September 2019 |
| | | GB 2572125 B | 13 January 2021 |
| | | IL 275703 A | 31 August 2020 |
| | | JP 7404242 B2 | 25 December 2023 |
| | | KR 10-2020-0106169 A | 11 September 2020 |
| | | US 2021-0059949 A1 | 04 March 2021 |
| | | WO 2019-135076 A1 | 11 July 2019 |
| KR 10-2020-0106049 A | 10 September 2020 | CA 3087802 A1 | 11 July 2019 |
| | | CN 111787910 A | 16 October 2020 |
| | | CN 111787910 B | 11 August 2023 |
| | | EP 3735231 A1 | 11 November 2020 |
| | | GB 2572126 A | 25 September 2019 |
| | | GB 2572126 B | 13 January 2021 |
| | | IL 275704 A | 31 August 2020 |
| | | JP 2021-509408 A | 25 March 2021 |
| | | JP 7378402 B2 | 13 November 2023 |
| | | US 2021-0059976 A1 | 04 March 2021 |
| | | WO 2019-135077 A1 | 11 July 2019 |

Form PCT/ISA/210 (patent family annex) (July 2022)

## REFERENCES CITED IN THE DESCRIPTION

*This list of references cited by the applicant is for the reader’s convenience only. It does not form part of the European patent document. Even though great care has been taken in compiling the references, errors or omissions cannot be excluded and the EPO disclaims all liability in this regard.*

### Patent documents cited in the description


- KR 102107715 B1